## Europäisches Patentamt

## European Patent Office

## Office européen des brevets

(19)

(11) Veröffentlichungsnummer: **0 099 371 B1**

(12)

# EUROPÄISCHE PATENTSCHRIFT

(45) Veröffentlichungstag der Patentschrift:
**22.04.87**

(51) Int. Cl.⁴: **A 61 B 17/16,** A 61 B 10/00

(21) Anmeldenummer: **83900339.9**

(22) Anmeldetag: **17.01.83**

(86) Internationale Anmeldenummer:
**PCT/EP 83/00007**

(87) Internationale Veröffentlichungsnummer:
**WO 83/02553 (04.08.83** Gazette **83/18)**

(54) **Instrument zur Vorbereitung der Entnahme von Knochenmaterial und Entnahme-Hohlzylinder.**

(30) Priorität: **25.01.82 DE 3202193**

(43) Veröffentlichungstag der Anmeldung:
**01.02.84 Patentblatt 84/5**

(45) Bekanntmachung des Hinweises auf die Patenterteilung:
**22.04.87 Patentblatt 87/17**

(84) Benannte Vertragsstaaten:
**AT BE CH DE FR GB LI NL SE**

(56) Entgegenhaltungen:
**DE - A - 2 258 069**
**DE - A - 3 007 036**
**FR - A - 1 052 375**
**FR - A - 2 419 060**
**US - A - 3 384 085**
**US - A - 3 683 892**

**Electromedica, Vol. 44, no. 3, 1976, Kirschner et al.:
"Innengekühlte Bohrer und Fräsen für die
Knochenchirugie in der Zahn-, Mund- und
Kieferheilkunde", pages 110-114**

(73) Patentinhaber: **DRAENERT, Klaus, Dr.med.,
Gabriel-Maxstrasse 3, D-8000 München 90 (DE)**

(72) Erfinder: **DRAENERT, Klaus, Dr.med.,
Gabriel-Maxstrasse 3, D-8000 München 90 (DE)**

(74) Vertreter: **Vossius & Partner, Siebertstrasse 4 P.O.
Box 86 07 67, D-8000 München 86 (DE)**

EP 0 099 371 B1

## Beschreibung

Die Erfindung betrifft ein Instrument zur Vorbereitung der Entnahme von Knochenmaterial und zur Vorbereitung einer Implantatbettes, das nach dem Schleifprinzip arbeitet, sowie einen Entnahme-Hohlzylinder zur Entnahme des mit diesem Instrument vorbereiteten Knochenmaterials.

Zur Entnahme von Knochenbiopsien und von Knochentransplantaten sowie zur Vorbereitung eines Implantatbettes werden bisher Knochenfräsen benutzt, die als schneidende, spanabhebende Bohrer oder Hohlbohrer mit scharfgezahnter Stirnfläche, wie die sogenannte Burkhardt-Fräse, gestaltet sind. Diese Fräsen besitzen erhebliche Nachteile, da sie zu erheblichen Weichteilzerreisungen bzw. Schnittverletzungen und Zerfetzungen führen, falls sie beim Arbeiten in die Weichteilgewebe abrutschen. Die als Hohlbohrer ausgebildeten Knochenfräsen erhalten zwar den Knochenzylinder, richten aber im Entnahmebett Zerstörungen an. Die als Bohrer ausgebildeten Fräsen erhalten das Entnahmebett, zerstören jedoch den Entnahmezylinder.

Die Knochenstruktur der mit solchen Hohlbohrern entnommenen Proben zeigt vielfache Verletzungen, da der Knochen geschnitten und teilweise gebrochen und zersplittert wird. Das Markgewebe wird in den Randpartien verletzt und ebenso wie Blutgefässe herausgerissen, so dass die morphologische Struktur der Biopsiezylinder in den Randzonen oft stark zerstört ist. Entsprechende Zerstörungen treten auch in den Randzonen eines durch einen Bohrer hergestellten Entnahmebetts auf und erschweren die Einheilung von Implantaten.

Das aus der US-A 3 384 085 bekannte chirurgische Schneidewerkzeug arbeitet wie zahnärztliche Bohrinstrumente mit einer sehr hohen Umdrehungsgeschwindigkeit von mehr als 100 000 min$^{-1}$. Die Werkzeugköpfe sind jedoch wie bei anderen bekannten Fräsern und Bohrern mit scharfen Kanten ausgebildet und wirken daher nicht nur schneidend, sondern auch zerreissend. Ausserdem ist es bei tiefer in den Knochen eindringenden Werkzeugen auch nicht mehr möglich, die durch die sehr rasche Umdrehung auftretende Reibungswärme genügend abzuführen, so dass Gewebeschädigungen durch Überhitzung eintreten.

Aus der DE-C 808 360 ist eine nicht näher beschriebene Vorrichtung zum Abtragen von Knochensubstanz bekannt, die aus einem mit kleinen, spitzen, glatten Zacken belegten Rotationskörper, vorzugsweise mit Abzugskanälen, besteht. Mit diesem Rotationskörper ist jedoch lediglich ein spanabhebendes Fräsen der Knochensubstanz unter Bildung von Knochenmus möglich. Die dabei auftretende Temperatur führt zu Knochennekrosen und zu nekrotischem denaturiertem Gewebsbrei.

Der Erfindung liegt die Aufgabe zugrunde, eine Vorrichtung zu schaffen, welche die Vorbereitung und Entnahme von Knochenzylindern (Biopsien) mit unzerstörter Randstruktur und die Herstellung von Implantatbetten mit unzerstörter Randstruktur ermöglicht und die beim Abgleiten keine Weichteilzerreissungen hervorrufen kann.

Es wurde nun gefunden, dass die Lösung der gestellten Aufgabe möglich ist, wenn man das Instrument als Schleifwerkzeug ausbildet. Es ist ausserdem vorteilhaft, dass man während des Schleifvorganges für eine Wärmeabfuhr durch Zufuhr einer Kühlflüssigkeit sorgt.

Zur Lösung der gestellten Aufgabe dienen erfindungsgemäss Instrumente gemäss Anspruch 1. Zur Entnahme des Knochenmaterials wurde erfindungsgemäss ein Entnahme-Hohlzylinder gemäss Anspruch 7 geschaffen.

Die Erfindung wird erläutert durch die folgende Beschreibung von Ausführungsbeispielen, die sich auf die beigefügten Zeichnungen beziehen. Hierin zeigen:

Fig. 1 einen schematischen Längsschnitt des Instruments,

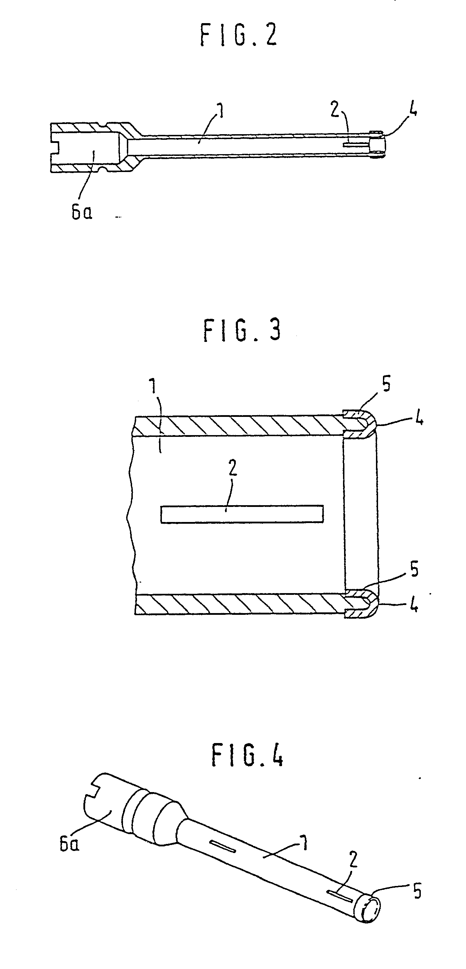

Fig. 2 einen schematischen Längsschnitt durch den Hohlzylinder des Instruments,

Fig. 3 Vergrösserung der Stirnseite des Hohlzylinders im Längsschnitt,

Fig. 4 Ansicht des auswechselbaren Hohlzylinders,

Fig. 5 einen schematischen Längsschnitt eines Entnahme-Hohlzylinders zur Entnahme von mit dem erfindungsgemässen Instrument vorbereitetem zylinderförmigem Knochenmaterial,

Fig. 6 einen Querschnitt durch den Entnahme-Hohlzylinder,

Fig. 7 Ansicht eines mit Handgriff ausgestatteten Entnahme-Hohlzylinders.

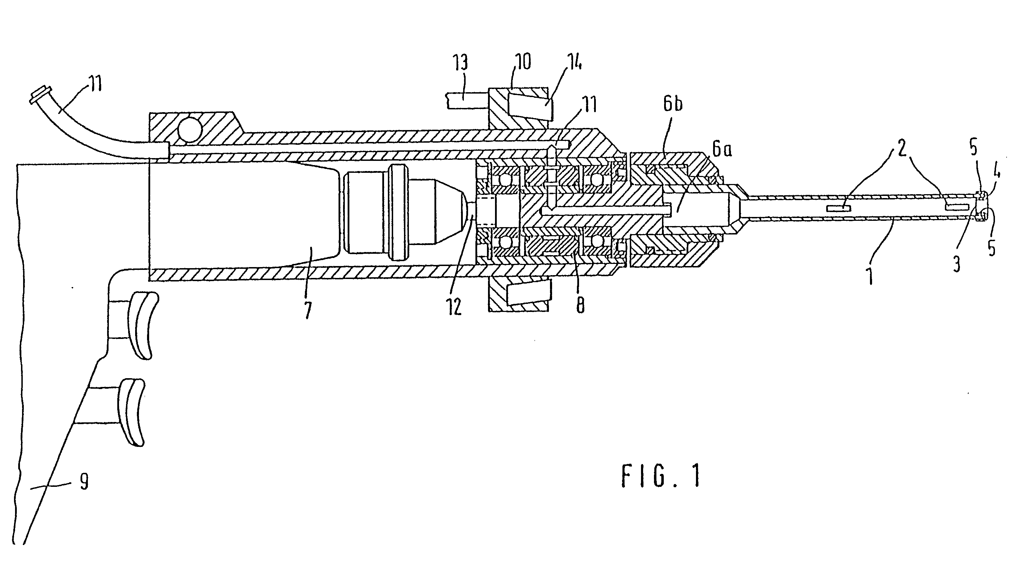

Das in Fig. 1 gezeigte chirurgische Instrument zeigt als Schleifwerkzeug einen länglichen Hohlzylinder 1. Dieser weist an einem Ende einen Kupplungsteil 6a auf, der mit dem Kupplungsteil 6b des schematisch dargestellten Antriebsteils 7 verbunden werden kann, durch welchen der Hohlzylinder 1 rotierend mit einstellbarer Geschwindigkeit angetrieben werden kann. Die Stirnfläche des Hohlzylinders 1 ist mit 4 bezeichnet. Der Innenquerschnitt des Hohlzylinders 1 nimmt von der Stirnfläche 4 bis zum Ausgang des Kupplungsteils 6a gleichmässig und geringfügig zu, um das Herausdrücken des erbohrten Knochenzylinders zu erleichtern. Im vorderen Bereich, nahe der Stirnfläche 4, weist der Hohlzylinder 1 Längsschlitze 2 auf, die vorzugsweise axial verlaufen.

Diese Längsschlitze 2 ermöglichen einen Abfluss der zugeführten Kühlflüssigkeit.

In dem den Schleifkopf 3 bildenden vorderen Bereich des Hohlzylinders 1, also vorzugsweise zwischen den Längsschlitzen 2 und der Stirnfläche 4, trägt der Hohlzylinder 1 auf seiner Innenwand und/oder Aussenwand einen dauerhaften Schleifbelag 5; die Stirnfläche 4 ist abgerundet, so dass eine grösstmögliche Beschichtungsoberfläche in Kontakt zum Knochen kommt.

Der Hohlzylinder 1 wird vorzugsweise aus rostfreiem Stahl gefertigt, der in der Regel in hochglanzpolierter Form verwendet wird, wie er für Präzisionsinstrumente üblich ist. Die Wandstärke des Hohlzylinders 1 variiert in Abhängigkeit von seinem Durchmesser, liegt aber normalerweise zwischen 0,09 und 0,90 mm, vorzugsweise zwischen 0,1 und 0,5 mm. Mindestens innen sind die Hohlzylinder leicht konisch gestaltet. Zweckmässig besitzen sie innen einen Freiwinkel von 5″ bis zu 0,5°, vorzugsweise 20″ bis 0,2°.

Der Schleifbelag 5 besitzt eine Schichtdicke von etwa 100 bis 700 µm, vorzugsweise bis 500 µm. Es kann aus üblichem, widerstandfähigem Material bestehen, z.B. Diamant, Korund, Aluminiumoxid-Keramik, Glas, Borazon oder vergleichbar harten Materialien. Die Korngrösse wird auf den Durchmesser des Schleifkopfes sowie auf die Art des zu bohrenden Knochens abgestimmt und beträgt 30 bis 350 µm, vorzugsweise bis 300 µm und zwar 50 bis 150 µm für Kompaktaknochen und um 150 µm für Spongiosaknochen. Eine Korngrösse um 50 µm ist für Schleifkopfdurchmesser von etwa 2 bis 8 mm besonders geeignet; eine solche von etwa 80 µm für Durchmesser von etwa 8 bis 12 mm und eine von 100 µm für Durchmesser von über 20 mm.

Bei einer Diamantbeschichtung liegt die Korngrösse vorzugsweise zwischen 30 und 250, maximal bei 350 µm.

Der Belag 5 wird in üblicher Weise aufgebracht, z.B. durch galvanische Beschichtung oder durch direkte oder indirekte Besputterung. Bei den grossen Zylindern kann auch Bestückung in weicher Legierung vorgesehen werden.

Die Stirnfläche 4 der Hohlzylinder 1 soll rund sein, damit ein Optimum an Schleifkörnern in Kontakt mit der zu schleifenden Knochensubstanz kommt.

Die Schlitze 2 in den Hohlzylindern 1 richten sich in ihrer Anzahl nach der Grösse der Hohlzylinder, d.h. nach dem Innendurchmesser. Löcher haben einen Durchmesser zwischen 200 und 1000 µm, rechteckige Fenster eine Länge von 2 bis 6 mm bei 0,3 bis 0,8 mm Breite.

Durchmesser und Länge der Hohlzylinder 1 sind auf die Art des vorzunehmenden chirurgischen Eingriffs abgestimmt. Die kleinsten Zylinder eines solchen Satzes sind mit einer Diamantbestückung mit Korngrössen um 50 µm versehen und für Feinknochenbiopsien bestimmt, vorwiegend für Kompaktaknochen bzw. für Sternalpunktionen, Schädeldachbiopsien und Biopsien zur Beurteilung des Verlaufes der Knochenbruchheilung.

Der Hohlzylinder 1 ist mittels seines Kupplungsteiles 6a in einen entsprechenden Kupplungsteil 6b des Antriebsteils 7 einsetzbar. Der Antriebsteil 7 weist eine rotierende Dichtung 8 auf, in der die Kühlflüssigkeitszuleitung 11 mündet, sowie einen Antriebszapfen 12, an dem das Futter des Antriebskopfes der in den Antriebsteil 7 einsetzbaren Antriebsmaschine angreift. Als Antriebsmaschine kann eine übliche Bohrmaschine mit einem Griff 9 dienen. Der Kupplungsteil 6b ist im Antriebsteil drehbar und unverschiebbar gelagert, beispielsweise mittels eines Kunststoff- oder Kugellagers. Der Antriebsteil 7 kann vorzugsweise aus einer Leichtmetall-Legierung hergestellt sein und ist ebenfalls präzisionsgearbeitet.

Durch die Kühlmittelzuleitung 11 gelangt eine physiologische Lösung, wie physiologische Kochsalz- bzw. Ringerlösung oder geeignete adaptierte Lösungen in den Hohlzylinder 1, wo sie durch die Längsschlitze 2 austritt. Das besondere dieser zentralen Wasserführung besteht in dem Druckaufbau durch die Rotation des Schleifhohlzylinders. Dieser wird gesteuert durch die Schlitzanordnung des Zylinders. Die Wasserzuführung erfolgt mit rotierender Dichtung. Die Wasserspülung wird zentral angeordnet, was den Vorteil hat, dass sich im Hohlzylinder 1 ein Druck aufbaut und primär kein hoher Ausgangsdruck erforderlich ist. Es genügt eine sterile Infusionsflasche über dem Operationstisch in einer Höhe zwischen 50 cm und 120 cm. Die Kühlmittelleitung ist mit einem (nicht gezeigten) Rückschlagventil versehen, das bei stehender Bohrmaschine den Kühlmittelzulauf unterbricht.

Ferner ist auf dem Antriebsteil 7 eine gegebenenfalls abnehmbare Beleuchtungseinrichtung 10 vorgesehen, welche auf den Schleifkopf 3 fokussierte Lichtstrahlen liefert. Die Beleuchtungseinrichtung kann beispielsweise zur Lichtzuleitung ein Lichtleitfaserbündel 13 aufweisen, welches das Licht den Lichtaustrittsöffnungen 14 zuleitet. Wenn als Lichtleiterfasern Quarzfasern verwendet werden, kann auch UV-Licht übertragen werden, um z.B. tetracyclinmarkierte Knochenbiopsien (bei Tumoren) oder fluoreszierende Kunststoffreste (bei Prothesenwechsel) besser sichtbar zu machen.

Zusätzlich zu dieser ringförmigen, aufgeschraubten bzw. fest angebrachten Mehrpunkt-Glasfaserbeleuchtung ist bei grossen Instrumenten, wie sie zu Vorbereitung der Markhöhle beim Prothesenwechsel Verwendung finden, zusätzlich ein zentraler Glasfaserlichtstab vorgesehen, der auf den Schleifkopf 3 fokussiert ist.

Zur Überpflanzung von Knorpelknochen und Knochentransplantaten sind sogenannte Zwillingshohlzylinder vorgesehen, d.h. ein Satz von zwei Hohlzylindern gleicher Länge, deren Durchmesser sich um 0,1 bis 0,4 mm unterscheidet, so dass der entnommene Knochenzylinder in ein 0,1 bis 0,4 mm kleineres Empfängerbett eingesetzt werden kann. Da vor allem Spongiosaknochen etwas elastisch sind und beim Einsetzen leicht komprimiert werden können, wird auf diese Weise eine stabile Implantation ohne Fremdmaterialien gewährleistet. Die Grösse des Unterschiedes der Durchmesser ist direkt abhängig vom Durchmesser des Hohlzylinders. Die Hohlzylinder von 4,5–8,5 mm Innendurchmesser unterscheiden sich um 0,2, kleinere um 0,1, grössere von 10 bis 35 mm Durchmesser um 0,2 bzw. max. 0,4 mm. Zwillingshohlzylinder für den Kompaktaknochen unterscheiden sich um 0,05 bis 0,1 mm. Die Zylinder werden jeweils nach der Beschichtung vermessen und einander zugeordnet.

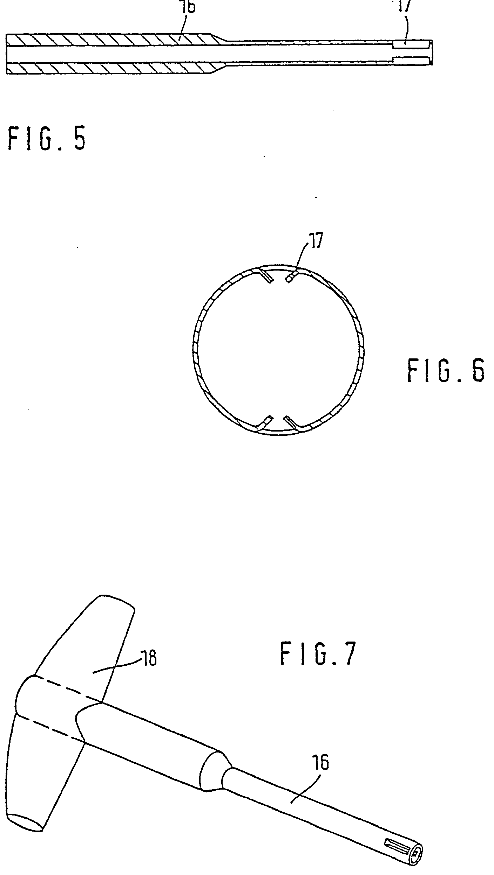

Zur Entnahme des durch den Hohlzylinder 1 beim Bohren erzeugten Knochenkerns dient vorzugsweise ein Entnahme-Hohlzylinder, beispielhaft in Fig. 5 gezeigt. Dieser Entnahme-Hohlzylinder 16 gleicht hinsichtlich der Wandstärke dem jeweiligen Schleif-Hohlzylinder, ist aber nicht aussen mit einem Schleifbelag versehen, sondern inwandig mit Mitnehmern 17, z.B. einem bis zehn feinen Widerhaken. Vorzugsweise sind diese im Querschnittsprofil der Entnahme-Hohlzylinder (Fig. 6) dreikantig. Sie können z.B. aufgelötet oder eingestanzt sein. Der Entnahme-Hohlzylinder wird nach Herausziehen des Schleif-Hohlzylinders über den stehengebliebenen Knochenkern vorsichtig eingeschlagen und dann leicht gedreht, wodurch der Knochenkern an seinem Fuss abgedreht wird und herausgezogen werden kann, da sich die Widerhaken des Entnahmezylinders im Knochengebälk bzw. in der Knochensubstanz verhaken. Der Knochenzylinder kann dann aus dem Entnahme-Hohlzylinder ohne Schwierigkeiten nach hinten ausgeschlagen werden. Vorzugsweise wird der Entnahme-Hohlzylinder 16 mit einem Handgriff 18 versehen (vgl. Fig. 7).

Die erfindungsgemässen Instrumente können auch mit Vorteil zur Vorbereitung des Implantatbettes benutzt werden. Der Vorteil liegt darin, dass das erfindungsgemässe Instrument einen feststehenden Knochenzylinder belässt, sodass das Implantat lediglich Knochensubstanz entsprechend der Wandstärke des Schleif-Hohlzylinders verdrängt. Sind die Implantate durchlöchert, so bedarf es nur einer geringen knöchernen Regeneration, um zu einer festen knöchernen Durchwachsung des Implantats zu führen. Die Instrumente für diesen Zweck weisen in einigen Fällen flexible Bohrwellen auf, um die erforderlichen Löcher einbringen zu können, z.B. bei Verankerung einer künstlichen Pfanne im Pfannendach, zur Biopsieentnahme auf der Innen- und Aussenseite des Beckenkammes und zur Vorbereitung des Implantatbettes bei Zahnersatz. Auf diese Weise erübrigt sich ein Winkelstück und es ist gleichwohl die zentrale Wasserspülung möglich.

Die erfindungsgemässen Instrumente werden durch spezielle Bohrführungen, die mit spitzen Zähnen versehen sind, geführt, wodurch Bohrmaschine und Schleifinstrument unverrückbar am Entnahmeort des Knochens gehalten werden. Für die flexiblen Wellen sind spezielle Halter notwendig, mit denen die gebogene Welle fest am Schleifpunkt gehalten werden kann.

Instrumente für bestimmte Entnahmestellen, z.B. Beckenkamm oder Sternum (Brustbein), können mit einem verstellbaren oder festen Anschlag versehen sein, sodass Zylinder definierter Länge entnommen werden können.

Zum Antrieb der erfindungsgemässen Instrumente eignen sich alle im Handel befindlichen Bohrmaschinen mit Drehzahlen zwischen 500 und 5000 min⁻¹. Höher drehende Bohrmaschinen sollten mit einem Reduktionsgetriebe auf niedere Tourenzahlen gebracht werden, da sonst die Gefahr besteht, dass die Kühlung durch die Kühlflüssigkeit nicht mehr ausreicht.

Die Bohrführungen sind vorzugsweise innen mit einer Kunststoffschicht versehen, damit der Schleifbelag der Hohlzylinder nicht durch die Bohrführung beschädigt wird. Da leichte Verkantungen möglich sind, wäre dies bei reiner Metallausführung möglich. Auch wenn die mit einem Schleifbelag versehenen Hohlzylinder bei vorsichtigem Aufsetzen in keiner Weise abdrehen oder weglaufen, wie dies von den scharfgezahnten Fräsköpfen bekannt ist, ist für den ungeübten Benutzer eine Bohrführung, vor allem auch für die flexiblen Wellen, vorgesehen, welche mit spitzen Zähnen bzw. wenigen Nadelspitzen die Hohlzylinder unverrückbar fest am Entnahmeort festhalten.

Der Anschluss an die chirurgischen Bohrmaschinen erfolgt über die Kupplungsteile, die die Vorrichtungen für die zentrale Spülung, die Dichtung und ein Schnellfutter zur Aufnahme der Hohlzylinder und der gegebenenfalls benötigten Übersetzungsstücke enthalten.

## Patentansprüche

1. Instrument zur Vorbereitung der Entnahme von Knochenmaterial sowie zur Vorbereitung eines Implantatbettes, mit einem rotierenden Hohlzylinder (C1), der über eine Kupplung (6a, 6b) lösbar mit einem Antriebsteil (7) verbunden ist, und an seinem vorderen Bereich zum Trennen des Knochenmaterials ausgebildet ist, dadurch gekennzeichnet, dass der Hohlzylinder (1) in seinem vorderen Bereich als Schleifkopf (3) mit abgerundeter Stirnfläche (4) ausgebildet ist und an der Aussen- und/oder Innenwand im Bereich des Schleifkopfes (3) mit einem Schleifbelag (5) versehen ist, dessen Partikelgrösse zwischen 30 und 350 µm liegt.

2. Instrument nach Anspruch 1, dadurch gekennzeichnet, dass der Hohlzylinder (1) leicht konisch gestaltet ist, wobei der Querschnitt zur Stirnfläche (4) hin abnimmt.

3. Instrument nach den Ansprüchen 1 und 2, gekennzeichnet durch eine innen im Hohlzylinder (1) mündende Kühlflüssigkeitszuleitung (11).

4. Instrument nach den Ansprüchen 1 bis 3, dadurch gekennzeichnet, dass der Hohlzylinder (1) mindestens eine Öffnung, vorzugsweise in Form von Schlitzen (2), besitzt, die vor der Stirnfläche (4) endet bzw. enden.

5. Instrument nach den Ansprüchen 1 bis 4, dadurch gekennzeichnet, dass am Antriebsteil (7) oder am Adapter zu einer das Antriebsteil antreibenden Bohrmaschine eine auf den Schleifkopf (3) gerichtete Beleuchtungseinrichtung (10) vorgesehen ist, vorzugsweise eine Glasfaser- bzw. Quarzfaserbeleuchtung, deren Lichtkegel auf die Spitze des Hohlzylinders fokussiert ist.

6. Instrumente nach den Ansprüchen 1 bis 5, dadurch gekennzeichnet, dass sie als Zwillingspaar gestaltet sind, indem der Aussendurchmesser eines Hohlzylinders (1) einen 0,1 bis 0,4 mm

geringeren Durchmesser aufweist als der Innendurchmesser des anderen Hohlzylinders.

7. Entnahme-Hohlzylinder (16) zur Entnahme von zylinderförmigen Knochenmaterial, das mit einem Instrument nach den Ansprüchen 1 bis 5 vorbereitet wurde, dadurch gekennzeichnet, dass der Entnahme-Hohlzylinder (16) den gleichen Innen- und Aussendurchmesser hat wie der entsprechende Hohlzylinder (1) des Instruments und mindestens einen Mitnehmer (17) an seiner Innenwand aufweist, der beim Aufschieben des Entnahme-Hohlzylinders (16) über das Knochenmaterial in dieses eindringt und es beim Drehen und folgenden Herausziehen des Entnahme-Hohlzylinders mitnimmt.

## Claims

1. Instrument for preparing the removal of bone material and for preparing an implant bed by means of a rotating hollow cylinder (1) which is joined, releasably via a coupling (6a, 6b), to a driving part (7), the front region of which is designed to remove the bone material characterised in that the front region of the hollow cylinder (1) is designed as a grinding head (3) with a rounded end face (4) and is provided on the outer and/or inner wall in the region of the grinding head (3) with an abrasive coating (5), the particle size of which is between 30 and 350 μm.

2. Instrument according to claim 1, characterised in that the hollow cylinder (1) has a slightly conical shape, the cross-section decreasing towards the end face (4).

3. Instrument according to claims 1 and 2, characterised by a coolant feed line (11) leading into the interior of the hollow cylinder (1).

4. Instrument according to claims 1 to 3, characterised in that the hollow cylinder (1) has at least one aperture, preferably in the form of slots (2) which end or ends before the end face (4).

5. Instrument according to claims 1 to 4, characterised in that, on the driving part (7) or on the adapter towards a drill which drives the driving part, an illumination device (10), preferably a glass fibre or quartz fibre illumination, is provided which points to the grinding head (3) and the light cone of which is focused on the tip of the hollow cylinder.

6. Instruments according to claims 1 to 5, characterised in that they are designed as a pair of twins, the external diameter of one hollow cylinder (1) being 0.1 to 0.4 mm smaller than the internal diameter of the other hollow cylinder.

7. Hollow removal cylinder (16) for the removal of cylindrical bone material which has been prepared with an instrument according to claims 1 to 5, characterised in that the hollow removal cylinder (16) has the same internal and external diameter as the corresponding hollow cylinder (1) of the instrument and that it has at least one tang (17) on the inside which, when the hollow removal cylinder (16) is pushed over the bone material, penetrates into said material and takes it along when the hollow removal cylinder is rotated and subsequently withdrawn.

## Revendications

1. Instrument pour préparer le prélèvement d'une matière osseuse ainsi que pour apprêter un lit d'implant, comportant un cylindre creux rotatif (1) qui est relié amoviblement à une partie d'entraînement (7) par l'intermédiaire d'un accouplement (6a, 6b) et est conçu, dans sa région antérieure, pour séparer la matière osseuse, caractérisé par le fait que le cylindre creux (1) est réalisé, dans sa région antérieure, sous la forme d'une tête de meulage (3) ayant une face frontale arrondie (4) et est pourvu sur sa paroi externe et/ou interne, au voisinage de la tête de meulage (3), d'une garniture de meulage (5) dont la grosseur de particules est comprise entre 30 et 350 μm.

2. Instrument selon la revendication 1, caractérisé par le fait que le cylindre creux (1) est de configuration légèrement conique, la section décroissant en direction de la face frontale (4).

3. Instrument selon les revendications 1 et 2, caractérisé par un conduit (11) d'admission d'un fluide de refroidissement, débouchant à l'intérieur du cylindre creux (1).

4. Instrument selon les revendications 1 à 3, caractérisé par le fait que le cylindre creux (1) possède au moins un orifice, de préférence sous la forme de fentes (2), qui s'achève(nt) avant la face frontale (4).

5. Instrument selon les revendications 1 à 4, caractérisé par le fait qu'il est prévu, sur la partie d'entraînement (7) ou sur l'adaptateur à une perceuse menant la partie d'entraînement, un dispositif d'éclairage (10) dirigé vers la tête de meulage (3), de préférence un éclairage par fibres de verre ou par fibres de quartz, respectivement, dont le cône de lumière est focalisé sur la pointe du cylindre creux.

6. Instruments selon les revendications 1 à 5, caractérisés par le fait qu'ils sont conçus sous la forme d'une paire jumelée, le diamètre externe d'un cylindre creux (1) présentant un diamètre inférieur de 0,1 à 0,4 mm au diamètre interne de l'autre cylindre creux.

7. Cylindre préleveur creux (16) pour prélever une matière osseuse en forme de cylindre ayant été préparée à l'aide d'un instrument selon les revendications 1 à 5, caractérisé par le fait que le cylindre préleveur creux (16) présente le même diamètre interne et externe que le cylindre creux correspondant (1) de l'instrument et comporte, sur sa paroi interne, au moins un organe d'entraînement (17) qui, lors de l'enfilement du cylindre préleveur creux (16) sur la matière osseuse, pénètre dans cette dernière et l'entraîne au cours de la rotation et de l'extraction consécutive du cylindre préleveur creux.

FIG. 1

# F I G . 2

# F I G . 3

# F I G . 4

16   17

## FIG. 5

17

## FIG. 6

18

## FIG. 7

16